# EUROPEAN PATENT APPLICATION

(11) **EP 2 221 004 A1**
(43) Date of publication of application: **25.08.2010**
(21) Application number: 09425072.7
(22) Date of filing: 24.02.2009
(51) Int. Cl.: A61B 6/14

(54) **Centering unit particularly for radiographic instruments**

(71) Applicant: Cirulli, Maurizio, 40136 Bologna (IT)
(72) Inventor: Cirulli, Maurizio, 40136 Bologna (IT)
(74) Representative: Modiano, Micaela Nadia

(57) **Abstract**

A centering unit (1), particularly for radiographic instruments, comprising a handle (2) of a supporting rod (3) for at least one insertion seat (4) of a radiographic head and for at least one receptacle (5) of a receiving lamina (6). The lamina (6) is designed to be struck by the radiographic beam emitted by the head. The seat (4) and the receptacle (5) are associated slidingly with the rod (3) by way of respective coupling means (7); in turn, the rod (3) is supported rotatably by the handle (2). Moreover, the rod (3) comprises, substantially at its centerline region, at least one substantially transverse appendage (8), which constitutes a support for a portion of the cranium of a patient (9) to be subjected to radiographic investigation.

## Description

The present invention relates to a centering unit, particularly for radiographic instruments.

As is known, the use of traditional radiological techniques, aimed at investigating the bony anatomical structures of the facial skeleton, of the mandibular structure and of the masticatory apparatus constituted by the dental elements, is widespread in the field of odontostomatology.

The acquired information is constituted by black and white images that show cross-sections of the organs struck by the radiation beam and thus allow to view two of the three dimensions (height and width) of the target.

Moreover, the different shades of gray with which the various organs are visualized can provide useful information regarding the higher or lower consistency of the bone systems being investigated.

With particular reference to prosthodontic procedures in the various mandibular and maxillary regions, this method of analysis allows to acquire data related to the structures of greater anatomical interest, such as the vasculonervous bundle of the lower alveolar canal with the mental foramina for the mandibular part and the structures of the connected antral recesses and sinuses for the maxillary part.

As noted, however, the acquired images provide no detail related to the third dimension (thickness) of the organs crossed by the radiation beam.

The study of bone thicknesses (which is necessary to perform a correct implant) is therefore entrusted to more complex techniques, such as OPT and teleradiographic explorations, or computer tomographies.

However, these methods are not free from drawbacks.

Orthopantomography (OPT) and teleradiography are used only for preoperative diagnostic cases, since they force the patient to a high radiogenic exposure.

In turn, computer tomography shows limitations that reduce its applicability substantially to activities for studying and planning the operation. It in fact requires complex and expensive instruments, which are seldom available in an ordinary dental surgery; moreover, the complexity of the operations required for computer tomography does not allow to repeat it many times, as is instead sometimes necessary due to specific requirements of the system to be installed.

In fact, if the surgeon wishes to perform several radiographic examinations, for example to avoid the danger of inserting screws with an incorrect inclination and/or arrangement (by analyzing the three-dimensional shape of the provided channel) or to check the consistency of the bone (gum) portion that will receive the implant, he cannot rely on the preventive results obtained by axial tomography.

It is therefore evident that even the most sophisticated investigation methods do not meet fully the needs of the surgeon during surgery, and that the surgeon is thus forced to use simple centering units of the known type. With such units, however, it is very difficult to maintain in the correct mutual position the radiographic apparatus and the receiving lamina with which they are provided, in order to repeat the analyses and thus obtain precious information regarding the thickness of the target.

The aim of the present invention is to solve the above-mentioned problems, by providing a centering unit that offers, in a practical and easy manner, the possibility to perform repeated radiographs, particularly of the stomatognathic system, to allow the simultaneous assessment of the height and thickness of the bony ridge or of the elements inserted in the bone system.

Within this aim, an object of the invention is to provide a centering unit that ensures the possibility to perform with great precision a sequence of radiographs of the organs of interest.

Another object of the invention is to provide a centering unit that allows to measure the dimensions of the implanted elements according to an appropriate distance measurement scale provided on such unit.

Another object of the invention is to provide a centering unit that ensures high reliability in operation.

Another object of the invention is to provide a centering unit that can be obtained easily starting from commonly commercially available elements and materials.

Another object of the invention is to provide a centering unit that has low costs and is safe in application.

This aim and these and other objects, that will become better apparent hereinafter, are achieved by a centering unit, particularly for radiographic instruments, which comprises a handle of a supporting rod for at least one insertion seat of a radiographic head and for at least one receptacle of a receiving lamina, which is designed to be struck by the radiographic beam emitted by the head, **characterized in that** said seat and said receptacle are associated slidingly with said rod by way of respective coupling means, said rod being supported rotatably by said handle and comprising, substantially at its centerline region, at least one substantially transverse appendage, which constitutes a support for a portion of the cranium of a patient to be subjected to radiographic investigation.

Further characteristics and advantages of the invention will become better apparent from the following detailed description of a preferred but not exclusive embodiment of the centering unit according to the invention, illustrated by way of non-limiting example in the accompanying drawings, wherein:

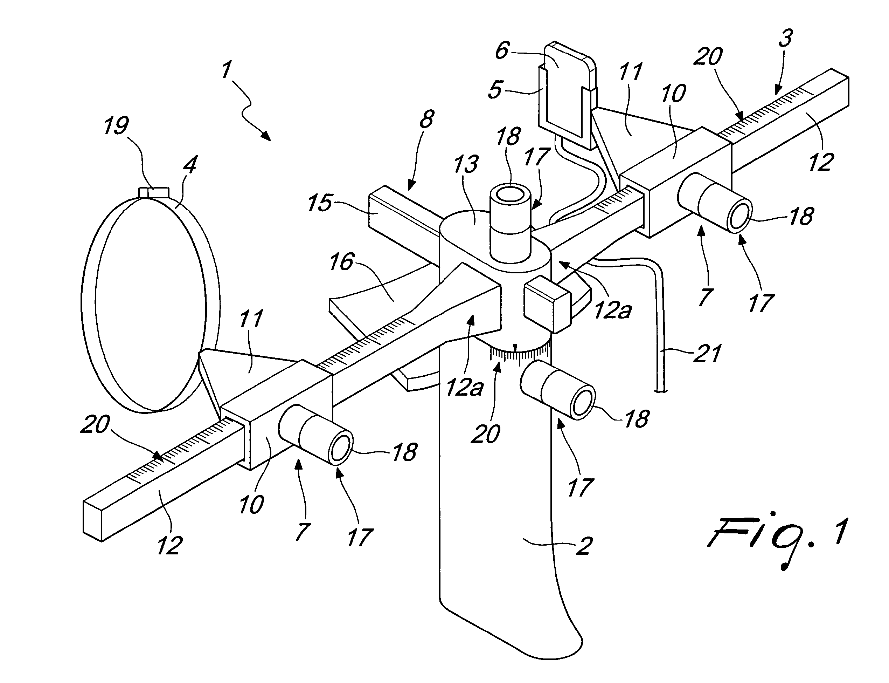

Figure 1 is a perspective view of a centering unit according to the invention;

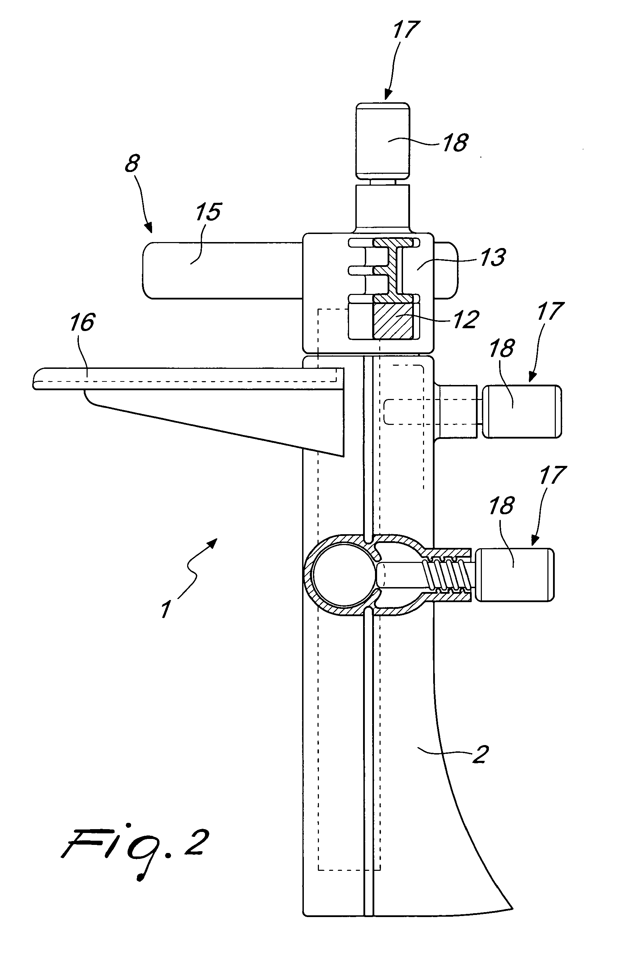

Figure 2 is a partially sectional side elevation view of a first detail of the unit according to the invention;

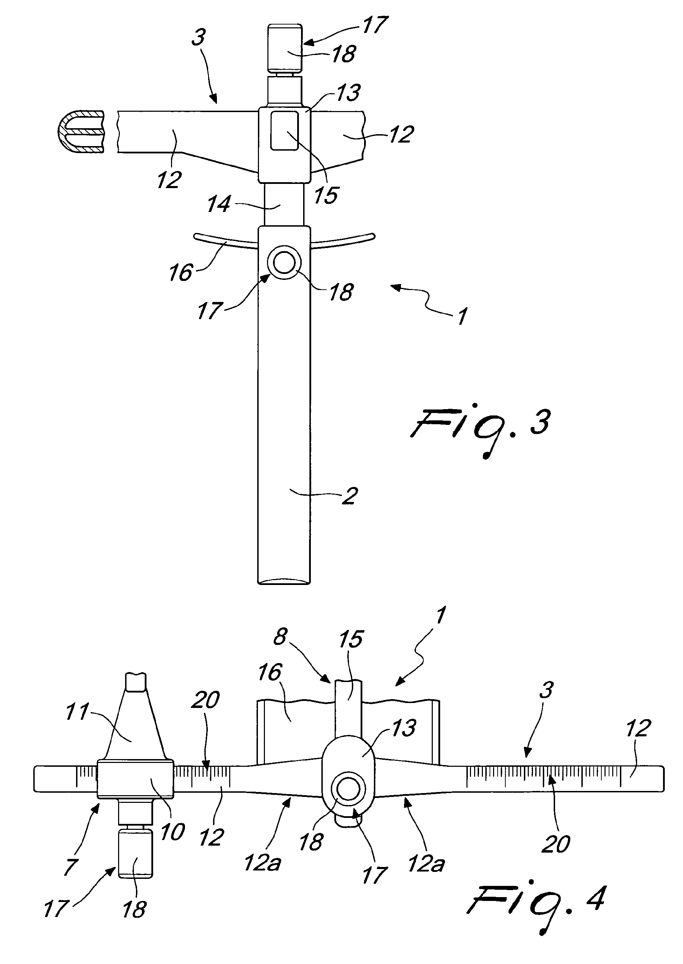

Figure 3 is a front elevation view of a second detail of the unit according to the invention;
Figure 4 is a top view of a third detail of the unit according to the invention;

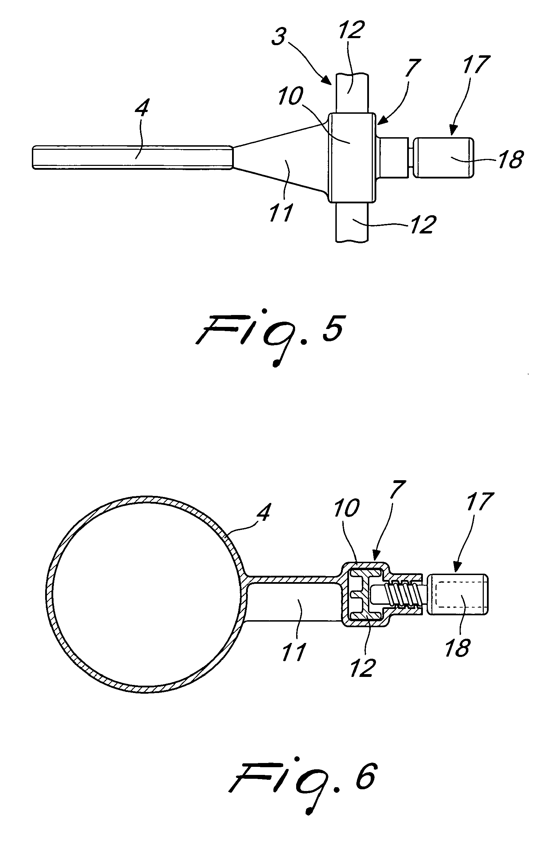

Figure 5 is a top view of a fourth detail of the unit according to the invention and shows the insertion seat of the radiographic head;
Figure 6 is a front elevation view of the same detail as Figure 5;

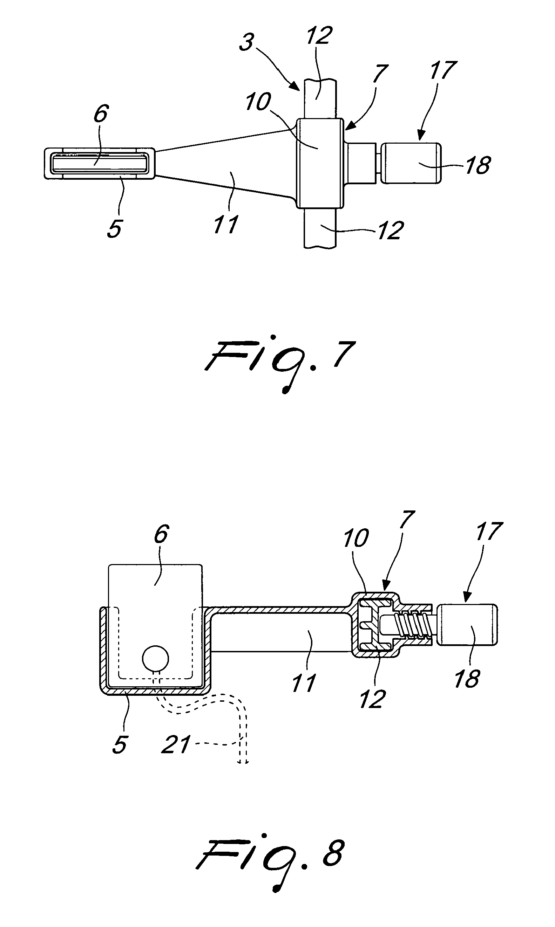

Figure 7 is a top view of a fifth detail of the unit according to the invention and shows the receptacle of the lamina;
Figure 8 is a front elevation view of the same detail as Figure 7;
Figure 9 is a side elevation view of the use of the unit according to the invention.

With reference to the figures, a centering unit according to the invention, generally designated by the reference numeral 1, comprises a handle 2 of a supporting rod 3 for at least one insertion seat 4 of a radiographic head and for at least one receptacle 5 of a receiving lamina 6.

The receiving lamina 6 is designed to be struck by the radiographic beam emitted by the head, and in greater detail a preferred application of the unit 1 according to the invention is constituted by its use during investigations performed with radiographic instruments, particularly for the field of dentistry.

However, it should be specified at the outset that different applications of the unit 1 according to the invention are not excluded if needed by the specific requirements.

According to the invention, the seat 4 and the receptacle 5 are associated slidingly with the rod 3 by way of respective coupling means 7; in turn, the rod 3 is supported so that it can rotate by the handle 2 and comprises, substantially at its centerline region, at least one substantially transverse appendage 8.

The appendage 8 constitutes a resting element for a portion of the cranium of a patient 9, such cranium being thus interposed between the radiographic head and the lamina 6, so as to subject the internal organs to radiographic investigation by displaying on the lamina 6 a radiographic image thereof (for example on a digital medium or on a photographic film comprised within the lamina 6).

More particularly, the coupling means 7 comprise respective sliders 10, which by means of suitable supporting ribs 11 support correspondingly in a rigid fashion the seat 4 and the receptacle 5; the sliders 10 can slide along the rod 3 on opposite sides with respect to the appendage 8.

In this manner, it is evident that it is possible to adjust the mutual distance of the radiographic head and of the lamina 6 as well as the distance of these two parts with respect to the cranium of the patient 9 according to the specific requirements of the surgeon.

Moreover, once a first investigation has been performed (and therefore information related to two of the three dimensions of the organs have been obtained), it is simple and easy to slide the sliders 10 in order to obtain one or more additional radiographic images, in order to allow to appreciate the third dimension (thickness) of the examined organs.

According to a solution of substantial practical interest, described by way of non-limiting example of the application of the invention, the rod 3 is substantially constituted by two arms 12 which are mutually aligned, and a respective slider 10 can slide on each one of such arms.

In any case, the possibility to provide rods 3 constituted by arms 12 that are not aligned but instead form for example a predefined angle is not excluded.

The connection between the two arms 12 is obtained by means of a block 13, to which each arm 12 is fixed at an end 12a thereof.

In turn, the block 13 is rigidly coupled to a stem 14 that can rotate in a cavity formed coaxially in the handle 2, in order to allow rotary coupling of the rod 3 with the handle 2.

The rotary coupling between the rod 3 and the handle 2 allows further possibilities to adjust the mutual arrangement of the various components of the unit 1 according to the invention, in order to better adapt it to the requirements and shape of the cranium of the patient 9. Further, as already noted previously for the possibility of the sliders 10 to slide, the rotary coupling also allows to vary the chosen configuration after a first radiographic investigation, in order to perform additional exams and thus obtain a more complete picture of the shape of the internal organs of the patient 9.

In order to give the unit 1 according to the invention further possibilities of adaptation, the appendage 8 comprises at least one bar 15, which can slide within the block 13, along a direction that lies transversely to the direction defined by the rod 3 (and by the radiographic beam), in order to allow adjustment of the resting contact of the patient 9.

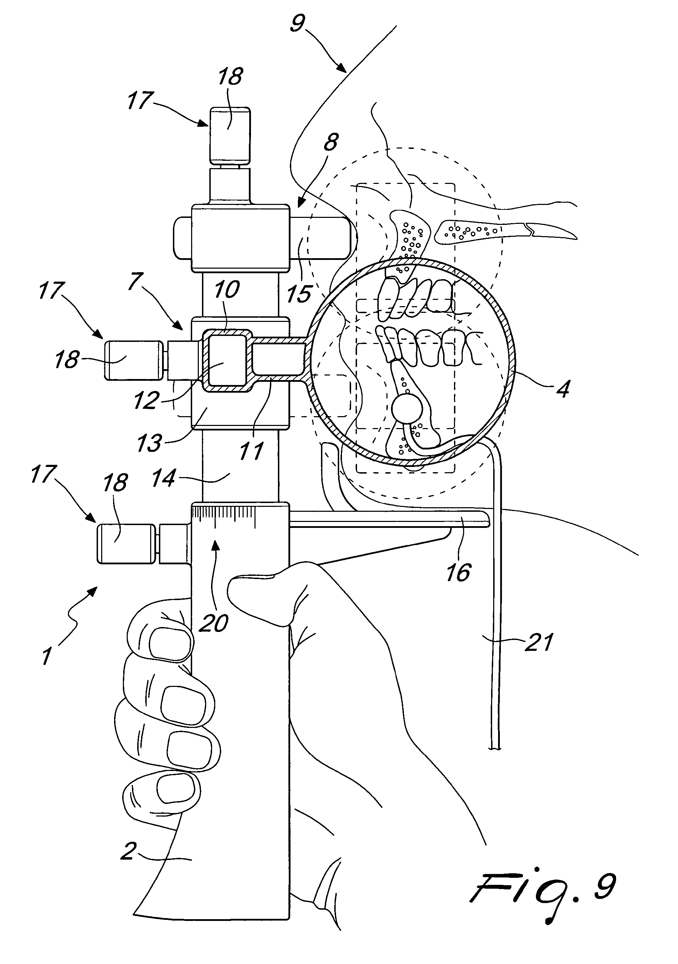

The bar 15 (optionally provided with a rounded or otherwise ergonomically shaped end portion) is designed to abut (as shown in Figure 9) against the forehead, nose or other region of the face of the patient 9 (for example the regions known as gonion and nasion).

In the solution shown in the accompanying figures, the unit 1 further has a plate 16, which is arranged below the bar 15 and on which the patient 9 can rest his/her chin (or which can be clamped between his/her teeth, according to various uses of the unit 1 according to the invention).

According to other embodiments, instead of the plate 16 the unit 1 can be provided with one or more rods, which are substantially parallel to the bar 15 and are again arranged at a lower level and in any case are designed to act as a support for the chin (or to be clamped between the teeth).

Once the chin has been rested on the plate 16, it is thus possible to adjust the position of the bar 15 in order to use it as reference and define and/or store the correct position of the head of the patient 9.

Conveniently, the unit 1 according to the invention comprises respective stop bodies 17, which are adapted to prevent the rotation of the rod 3 with respect to the handle 2 and are adapted to prevent the sliding of each slider 10 and of the bar 15 (respectively on the rod 3 and inside the block 13). This ensures that the desired configuration is maintained once it has been identified, particularly during radiographic investigation.

Each stop body 17 can thus be actuated to stably and mutually couple two of the components cited above, preventing respectively mutual sliding or rotation. More particularly, according to the solution shown in the accompanying figures, each body 17 comprises a screw 18, which can move by screwing inside one of the components in order to abut against a surface portion of the second component, blocking mutual movement by friction.

In order to eliminate the coupling thus obtained, it is sufficient to unscrew the screw 18, moving it in the opposite direction and thus moving it away from the surface portion of the second component.

Advantageously, the unit 1 according to the invention comprises a device 19 for pointing the radiographic beam, which can be functionally associated with the radiographic head, in order to facilitate the correct adjustment and mutual arrangement of the head and of the lamina 6, with the cranium of the patient 9 rested on the appendage 8.

In particular, the pointing device 19 is constituted by a laser pointer, which is fixed to the insertion seat 4 of the radiographic head and is oriented so as to visualize on the cranium of the patient 9 the direction of the radiographic beam (in the form of a small luminous circle).

According to a further embodiment, the centering unit 1 according to the invention comprises a motor apparatus, which is connected for example to the radiographic head and is designed to move the rod 3 in order to actuate, even according to predefined rules of motion, its rotation with respect to the handle 2.

The motorization of the rod 3 and the possibility to predefine the corresponding rule of motion allow for example to set beforehand a specific number of stops in preset angular positions of the rod 3, in which corresponding radiographic investigations are to be performed.

Conveniently, the unit 1 according to the invention comprises at least one graduated scale 20, which is provided on the outer surface of a least one between the rod 3, the appendage 8, the handle 2 and the block 13, in order to allow to identify their mutual position and any scale factor of the image that is the result of the radiographic investigation.

In the embodiment shown in the accompanying figures, the graduated scale 20 is imprinted on the arms 12 of the rod 3 (to measure the movements of the sliders 10 and therefore the mutual distance of the seat 4, of the receptacle 5 and of the cranium of the patient 9) and on the handle 2 (in order to measure the angle of rotation of the rod 3 with respect to the handle 2).

It should be noted in any case that the possibility to provide additional graduated scales 20 on other components of the unit 1 according to the invention is not excluded.

In order to allow the surgeon a more detailed visualization of the resulting radiographic images as well as their reprocessing, the unit 1 according to the invention may positively comprise means 21 for connection to a control and management apparatus, of the type of a personal computer provided with suitable software, which is adapted to receive and process the radiographic image that is imprinted on the lamina 6 and display it, even with high resolution, on a monitor.

This software can be for example capable of eliminating images related to what is arranged in front and/or behind the target of the analysis, thus obtaining an image that is clearer and without interference.

The use of the centering unit according to the invention is as follows.

The surgeon who needs to investigate the bone structures of the patient 9 (the upper and/or lower dental arch, for example) can easily resort to the unit 1, arranging the head of the patient 9 at the appendage 8 and at the plate 16, as already described.

Subsequently, while the patient 9 clamps the handle 2 between his/her fingers, the surgeon can arrange the various components of the unit 1 according to infinite different configurations, in order to obtain the mutual arrangement of the radiographic head, of the cranium of the patient 9 and of the lamina 6 that best allow him to visualize the organs in which he is interested.

As already observed, the simplicity of the movement of the various components, which can be subsequently locked by means of the stop bodies 17, gives assurance to the surgeon of the possibility to repeat the analysis several times, in order to obtain a substantially three-dimensional representation of the observed organs.

The infinite configurations that can be obtained allow to use the unit 1 according to the invention for a plurality of radiographic investigations.

For example, by means of the unit 1 it is possible to obtain anteroposterior projections (superior ones, for inclusions in the upper maxillary bone) or occlusal ones (inferior, intraoral maxillary or mandibular, right or left); further, by providing suitable simple modifications to the seat 4 of the radiographic head (in particular by containing its dimensions) it is possible to perform endo-oral radiographs.

Moreover, by way of non-limiting example, it is possible to investigate the inferior and superior frontal parasymphyseal sectors.

In practice it has been found that the centering unit according to the invention fully achieves the intended aim, since the possibility of sliding on the rod of the seat of the radiographic head and of the receptacle of the receiving lamina and the possibility of rotation of such rod with respect to the handle of the unit allow, in a practical and easy manner, to perform repeated radiographs, particularly of the stomatognathic system, to allow simultaneous assessment of the height and thickness of the bone ridge or of the elements inserted in the bone structure.

The invention thus conceived is susceptible of numerous modifications and variations, all which are within the scope the appended claims; all the details may further be replaced with other technically equivalent elements.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the dimensions, may be any according to requirements and to the state of the art; in particular, the unit 1 can be made of materials that can be disinfected and sterilized even in an autoclave.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A centering unit, particularly for radiographic instruments, comprising a handle (2) of a supporting rod (3) for at least one insertion seat (4) of a radiographic head and for at least one receptacle (5) of a receiving lamina (6), which is designed to be struck by the radiographic beam emitted by the head, **characterized in that** said seat (4) and said receptacle (5) are associated slidingly with said rod (3) by way of respective coupling means (7), said rod (3) being supported rotatably by said handle (2) and comprising, substantially at its centerline region, at least one substantially transverse appendage (8), which constitutes a support for a portion of the cranium of a patient (9) to be subjected to radiographic investigation.

2. The unit according to claim 1, **characterized in that** said coupling means (7) comprise respective sliders (10), which support rigidly and correspondingly said seat (4) and said receptacle (5), said sliders (10) being able to slide along said rod (3) on opposite sides with respect to said appendage (8).

3. The unit according to claims 1 and 2, **characterized in that** said rod (3) is constituted substantially by two mutually aligned arms (12), on each of which a respective slider (10) can slide, said arms (12) being fixed to each other at a block (13), which in turn is rigidly coupled to a stem (14) that can rotate within a cavity that is formed coaxially in said handle (2), in order to allow the rotary mating of said rod (3) with said handle (2).

4. The unit according to one or more of the preceding claims, **characterized in that** said appendage (8) comprises at least one bar (15), which can slide within said block (13) along a direction that lies transversely to the one formed by said rod (3), in order to allow adjustment of the resting contact of the patient (9).

5. The unit according to one or more of the preceding claims, **characterized in that** it comprises respective stop bodies (17), which are adapted to prevent the rotation of said rod (3) with respect to said handle (2) and are adapted to prevent the sliding of each one of said sliders (10) and of said bar (15) in order to maintain the desired configuration during radiographic investigation.

6. The unit according to one or more of the preceding claims, **characterized in that** it comprises a device (19) for pointing the radiographic beam, which is functionally associated with the radiographic head to facilitate the correct adjustment and mutual arrangement of the head and of said lamina (6), with the cranium of the patient (9) rested on said appendage (8).

7. The unit according to claim 6, **characterized in that** said pointing device (19) is constituted by a laser pointer, which is fixed to said seat (4) and is oriented so as to visualize on the cranium of the patient (9) the direction of the radiographic beam.

8. The unit according to one or more of the preceding claims, **characterized in that** it comprises a motor apparatus that is designed to move said rod (3) in order to actuate, even according to predefined rules of motion, its rotation with respect to said handle (2).

9. The unit according to one or more of the preceding claims, **characterized in that** it comprises at least one graduated scale (20), which is provided on the outer surface of at least one between said rod (3), said appendage (8), said handle (2) and said block (13), in order to identify their mutual position and any scale factor of the image that is the result of the radiographic investigation.

10. The unit according to one or more of the preceding claims, **characterized in that** it comprises means (21) for connection to a control and management apparatus that is adapted to receive the radiographic image imprinted on said lamina (6) and to display it on a monitor.
